# EUROPEAN PATENT SPECIFICATION

(11) **EP 4 766 804 B1**
(45) Date of publication and mention of the grant of the patent: **18.11.2026**
(21) Application number: 24758830.4
(22) Date of filing: 21.08.2024
(51) Int. Cl.: A61K 8/44, C11D 1/94, A61K 8/46, A61Q 19/10, C11D 1/10, C11D 1/12, C11D 1/14, C11D 1/90, C11D 1/92

(54) **CLEANSING COMPOSITION**
REINIGUNGSZUSAMMENSETZUNG
COMPOSITION DE NETTOYAGE

(30) Priority: 23.08.2023 EP 23193014
(43) Date of publication of application: 01.07.2026
(73) Proprietor: Unilever IP Holdings B.V., 6708 WH Wageningen (NL); UNILEVER GLOBAL IP LIMITED, Wirral Merseyside CH62 4ZD (GB)
(72) Inventor: CARNALI, Joseph Oreste, 6708 WH Wageningen (NL); HIBAN, Douglas John, 6708 WH Wageningen (NL); JANKOVSKY, Megan Ruth, 6708 WH Wageningen (NL); MOADDEL, Teanoosh, 6708 WH Wageningen (NL); POST, Albert Joseph, 6708 WH Wageningen (NL); SHAFER, Georgia L., 6708 WH Wageningen (NL)
(74) Representative: Unilever Patent Group
(86) International application number: PCT/EP2024/073464
(87) International publication number: WO 2025/040717

(56) References cited:
- WO-A1-2014/181342
- WO-A1-2018/206215
- WO-A1-2022/233624
- US-A1- 2022 211 600
- US-A1- 2023 049 117

## Description

### Field of the Invention

Disclosed herein is a cleansing composition. The cleansing composition comprises an anionic surfactant comprising a first anionic surfactant and a second anionic surfactant and a zwitterionic surfactant.

### Background of the Invention

Personal cleansing processes characteristically involve the use of surfactant comprising compositions (i.e., wash compositions) and water to topically generate consumer desired lather and to rinse off the compositions typically applied to the face, hands, hair, and/or body. Such activities are performed daily, often more than once a day, and especially, as it relates to the hands and face of a consumer. Ideally, washing can be optimized when using stable products that are mild, have excellent lather characteristics and that rinse off with relative ease.

Personal cleansing formulations for skin and hair have historically been based on some combination of surfactants which include sodium lauryl ether sulfate (SLES). SLES offers the advantages of being widely available, an effective foaming agent, and inexpensive. However, the universal dependence of the industry on SLES has also exposed a number of shortcomings.

Surfactants based on the sulfate headgroup are widely identified with washing off the skin and scalp's protective barrier, leading to redness, dryness, and itch. Long term use can lead to dermatitis and edema. Eye irritation is also an issue with SLES. For these reasons, SLES is judged to be harsh to skin and scalp and there is currently industry-wide interest in so-called sulfate free cleansing surfactants, meaning that sulfonate- and carboxylate-based surfactants which are mild to skin and scalp are under development.

The ether-component of SLES is also under scrutiny because products containing it can be contaminated with hundreds of ppm levels of 1,4-dioxane as a by-product of production. Recent regulations regarding 1,4-dioxane have made its removal from products which contact humans as being actively encouraged.

For all these reasons, there is a need for an effective, foaming agent which does not rely on a head group containing sulfate. Surfactant headgroups based on sodium methyl taurate and sodium isethionate are good examples of sulfate and 1,4-dioxane free alternatives which still provide surface activity and good foaming. Popular commercial examples of these surfactants include sodium methyl lauroyl taurate, sodium methyl cocoyl taurate, sodium lauroyl isethionate, and sodium cocoyl isethionate.

US 2022/211600 A1 discloses compositions suitable for cleansing, comprising sodium cocoyl isethionate or sodium luroyl isethionate.

It is continually desired to develop personal cleansing compositions and formulations that are mild to the skin and have excellent in-use properties, such as foaming.

### Summary of the Invention

Disclosed in various aspects are cleansing compositions.

According to the invention, the cleansing composition comprises: 3 to 20% by weight of a total amount of surfactant, preferably, 5 to 18% by weight of a total amount of surfactant, more preferably, 5 to 15% by weight of a total amount of surfactant, based on the total weight of the cleansing composition, wherein the surfactant comprises: an anionic surfactant comprising a first anionic surfactant comprising: 11 to 30% by weight C8 to C10 chains, preferably, 12 to 25% by weight C8 to C10 chains, more preferably, 13 to 20% by weight C8 to C10 chains, based on the total weight of the anionic surfactant; 70 to 89% by weight C12 to C14 chains, based on the total weight of the anionic surfactant; and less than or equal to 0.5% by weight C16 to C18 chains, based on the total weight of the anionic surfactant; and a zwitterionic surfactant; wherein a weight ratio of anionic surfactant to zwitterionic surfactant is 1:4 to 1:1, preferably wherein the weight ratio of anionic surfactant to zwitterionic surfactant is 1:2 to 2:3; and wherein the first anionic surfactant comprises isethionate. These and other features and characteristics are more particularly described below.

### Detailed Description of the Invention

Disclosed herein is a cleansing composition that comprises an anionic surfactant comprising a first anionic surfactant and a second anionic surfactant. The cleansing composition also comprises a zwitterionic surfactant. It was surprisingly found that the cleansing compositions disclosed herein can provide mild cleansing compositions that maybe substantially free or essentially free of sulfate. The cleansing compositions may also be substantially free or essentially free of 1,4-dioxane. The cleansing compositions may be sulfate free and 1,4-dioxane free.

It was unexpectedly found that by replacing a portion of the long chain carbons (i.e., C12-C14) with shorter chain carbons such as C8 and C10 or even C9 chains, in the cleansing compositions disclosed herein lather performance on parity with sulfate based cleansers could be achieved. For example, when using sodium lauroyl isethionate as the first anionic surfactant, it was unexpectedly found that replacing a portion of the C12 chains of the sodium lauroyl isethionate with C8, C10 isethionate or C9 isethionate, lather parity could be achieved.

Table 1 shows a typical chain breakdown for C9 isethionate and sodium lauroyl isethionate.

**Table 1: C9 and Sodium lauroyl isethionate chain breakdown**

| Isethionate | C6 | C8 | C9 | C10 | C12 | C14 | C16 | C18 |
|---|---|---|---|---|---|---|---|---|
| C9 Isethionate | 0% | 0% | 100% | 0% | 0% | 0% | 0% | 0% |
| Sodium lauroyl isethionate | 0.25% | 3% | 0% | 2% | 94% | 0.25% | 0.25% | 0.25% |

Replacing part of the C12 groups in the anionic surfactant can reduce reliance on palm kernel oil. For example, replacing a portion of the C12 chains in the anionic surfactant allows for a closer match to the natural distribution of chains in palm kernel oil, thereby allowing more efficient use of palm kernel oil.

Substantially free or essentially free as used herein refers to an amount of less than or equal to 100 parts per million in the cleansing composition, for example, less than 50 ppm in the cleansing composition, for example, less than or equal to 25 ppm in the cleansing composition, for example, less than or equal to 10 ppm in the cleansing composition, for example, less than or equal to 5 ppm in the cleansing composition, for example, less than or equal to 4 ppm in the cleansing composition, for example, less than or equal to 2 ppm in the cleansing composition, for example, less than or equal to 1 ppm in the cleansing composition, for example, less than or equal to 0.5 ppm in the cleansing composition.

The cleansing composition comprises 3 to 20% by weight of a total amount of surfactant, for example, 5 to 18% by weight of a total amount of surfactant, for example, 5 to 15% by weight of a total amount of surfactant, based on the total weight of the cleansing composition.

The surfactant comprises an anionic surfactant and a zwitterionic surfactant. The weight ratio of anionic surfactant to zwitterionic surfactant is 1:4 to 1:1, for example, the weight ratio can be 1:2 to 2:3. Total anionic surfactant may preferably be present in an amount of from 1 to 5 wt%, preferably from 2.5 to 5 wt%, based on the weight of the composition. The total amount of zwitterionic surfactant may preferably be from 2 to 15 wt%, preferably of from 4 to 8 wt%, based on the weight of the composition.

The anionic surfactant can comprise a first anionic surfactant and a second anionic surfactant.

The first anionic surfactant comprises 11 to 30% by weight C8 to C10 chains, preferably, 12 to 25% by weight C8 to C10 chains, more preferably, 13 to 20% by weight C8 to C10 chains, based on the total weight of the anionic surfactant. The first anionic surfactant comprises 70 to 89% by weight C12 to C14 chains, based on the total weight of the anionic surfactant and the first anionic surfactant can comprise less than or equal to 0.5% by weight C16 to C18 chains, based on the total weight of the anionic surfactant.

The C14 chains can be present in an amount of less than or equal to one half of the sum of C12 and C14 chains present in the first anionic surfactant, for example, the C14 chains can be present in an amount of less than or equal to one third of the sum of C12 and C14 chains present in the first anionic surfactant.

The first anionic surfactant can be present in an amount of greater than or equal to 85% by weight of the anionic surfactant, for example, greater than or equal to 90% by weight of the anionic surfactant, for example, greater than or equal to 95% by weight of the anionic surfactant, for example, greater than or equal to 97% by weight of the anionic surfactant, for example, greater than or equal to 99% by weight of the anionic surfactant.

The second anionic surfactant can be present in an amount of less than or equal to 15% by weight of the anionic surfactant, less than or equal 10% by weight of the anionic surfactant, for example, less than or equal to 5% by weight of the anionic surfactant, for example, less than or equal to 3% by weight of the anionic surfactant, for example, less than or equal to 1% by weight of the anionic surfactant.

In addition to the anionic surfactant the zwitterionic surfactant, the cleansing composition can also comprise a nonionic surfactant, an amphoteric surfactant, a cationic surfactant, or a combination thereof. The cleansing composition can contain the surfactant in a total amount of 3 to 20% by weight, preferably 5 to 18% by weight, more preferably 5 to 15% by weight, based on the total weight of the cleansing composition.

The first anionic surfactant comprises isethionate. The anionic surfactant may further include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate.

The anionic surfactant may also be alkyl sulfosuccinates (including mono- and dialkyl, e.g., C₆-C₂₂ sulfosuccinates); alkyl and acyl taurates (often methyl taurates), alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, and the like.

Sulfosuccinates may be monoalkyl sulfosuccinates having the formula:

R¹OC(O)CH₂CH(SO₃M)CO₂M;

and amide-MEA sulfosuccinates of the formula:

R¹CONHCH₂CH₂OC(O)CH₂CH(SO₃M)CO₂M

wherein R¹ ranges from C₈-C₂₂ alkyl.

Sarcosinates are generally indicated by the formula:

R²CON(CH₃)CH₂CO₂M,

wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described.

The cleansing composition disclosed herein contains C₈-C₁₈ acyl isethionates. These esters are prepared by a reaction between alkali metal isethionate with mixed aliphatic fatty acids having from 6 to 18 carbon atoms and an iodine value of less than 20. In an embodiment, at least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995. This compound has the general formula:

R⁵C-(O)O-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

According to the invention, the first anionic surfactant comprises isethionate. In an aspect of the cleansing composition, the first anionic surfactant is isethionate. The isethionate can be present in an amount of greater than or equal to 80% by weight of the anionic surfactant, for example, can be present in an amount of greater than or equal to 80% by weight of the anionic surfactant, for example, greater than or equal to 85% by weight of the anionic surfactant, for example, greater than or equal to 90% by weight of the anionic surfactant, for example, greater than or equal to 95% by weight of the anionic surfactant, for example, greater than or equal to 97% by weight of the anionic surfactant, for example, greater than or equal to 99% by weight of the anionic surfactant.

The anionic surfactant can optionally contain a second anionic surfactant. In an aspect the anionic surfactant contains a second anionic surfactant. The second anionic surfactant can be present in an amount of less than or equal to 20% by weight of the anionic surfactant, for example, less than or equal to 15% by weight of the anionic surfactant, less than or equal 10% by weight of the anionic surfactant, for example, less than or equal to 5% by weight of the anionic surfactant, for example, less than or equal to 3% by weight of the anionic surfactant, for example, less than or equal to 1% by weight of the anionic surfactant.

The second anionic surfactant can comprise sodium gluconate, sodium lauroyl glycinate, sodium lauroyl glutamate, sodium lauroyl isethionate, sodium lauryl sarcosinate, alpha olefin sulfonate (AOS), or a combination thereof.

Amphoteric surfactants can be included in the cleansing compositions disclosed herein. Amphoteric surfactants (which depending on pH can be zwitterionic) include sodium acyl amphoacetates, sodium acyl amphopropionates, disodium acyl amphodiacetates and disodium acyl amphodipropionates, where the acyl (i.e., alkanoyl group) can comprise a C₇-C₁₈ alkyl portion. Illustrative examples of amphoteric surfactants include sodium lauroamphoacetate, sodium cocoamphoacetate, sodium lauroamphoacetate, or a combination thereof.

As to the zwitterionic surfactants employed in the present cleansing composition, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms and generally comply with an overall structural formula:

R⁶-[-C(O)-NH(CH₂)_{q}-]ᵣ-N⁺(R⁷)(R⁸)-A-B

where R⁶ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is -CO₂- or -SO₃-.

Desirable zwitterionic surfactants for use in the cleansing composition disclosed herein and within the above general formula include simple betaines of formula:

R⁶-N⁺(R⁷)(R⁸)-CH₂CO₂⁻

and amido betaines of formula:

R⁶-CONH(CH₂)ₜ-N⁺ (R⁷)(R⁸)-CH₂CO₂⁻

where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of formula:

R⁶-N⁺(R⁷)(R⁸)-(CH₂)₃SO₃⁻ or

R⁶-CONH(CH₂)ᵤ-N⁺(R⁷)(R⁸)-(CH₂)₃SO₃⁻

where u is 2 or 3, or variants of these in which - (CH₂)₃SO₃⁻ is replaced by

-CH₂C(OH)(H)CH₂SO₃⁻.

In these formulae, R⁶, R⁷ and R⁸ are as previously defined.

Illustrative examples of the zwitterionic surfactants desirable for use include betaines such as lauryl betaine, betaine citrate, cocodimethyl carboxymethyl betaine, cocoamidopropyl betaine, coco alkyldimethyl betaine, and laurylamidopropyl betaine. An additional zwitterionic surfactant suitable for use includes cocoamidopropyl sultaine, for example, cocamidopropyl hydroxysultaine. Other desirable zwitterionic surfactants include lauryl betaine, betaine citrate, sodium hydroxymethylglycinate, (carboxymethyl) dimethyl-3-[(1-oxododecyl) amino] propylammonium hydroxide, coco alkyldimethyl betaine, (carboxymethyl) dimethyloleylammonium hydroxide, cocoamidopropyl betaine, (carboxymethyl) dimethyloleylammonium hydroxide, cocoamidopropyl betaine, (carboxylatomethyl) dimethyl(octadecyl)ammonium, cocamidopropyl hydroxysultaine, or a combination thereof.

In an embodiment, the zwitterionic surfactant can comprise lauryl betaine, cocobetaine, (carboxymethyl) dimethyl-3-[(1-oxododecyl) amino] propylammonium hydroxide, coco alkyldimethyl betaine, , (carboxymethyl) dimethyloleylammonium hydroxide, cocoamidopropyl betaine, (carboxylatomethyl) dimethyl(octadecyl)ammonium, cocamidopropyl hydroxysultaine, laurylamidopropyl betaine, lauryl hydroxysultaine, or a combination thereof.

In an embodiment, the zwitterionic surfactant is lauryl betaine, cocobetaine, (carboxymethyl) dimethyl-3-[(1-oxododecyl) amino] propylammonium hydroxide, coco alkyldimethyl betaine, , (carboxymethyl) dimethyloleylammonium hydroxide, cocoamidopropyl betaine, (carboxylatomethyl) dimethyl(octadecyl)ammonium, cocamidopropyl hydroxysultaine, laurylamidopropyl betaine, lauryl hydroxysultaine, or a combination thereof. Such surfactants are made commercially available from suppliers like Stepan Company, Solvay, Evonik and the like and it is within the scope of the cleansing compositions disclosed herein to employ mixtures of the aforementioned surfactants.

Nonionic surfactants may optionally be used in the cleansing composition. When used, nonionic surfactants are typically used at levels as low as 0.5, 1, 1.5 or 2% by weight and at levels as high as 6, 8, 10 or 12% by weight. The nonionic surfactants which can be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols, ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an aspect, nonionic surfactants can include fatty acid/alcohol ethoxylates having the following structures a) HOCH₂(CH₂)ₛ(CH₂CH₂O)_{c} H or b) HOOC(CH₂)ᵥ(CH₂CH₂O)_{d} H; where s and v are each independently an integer up to 18; and c and d are each independently an integer from 1 or greater. In an aspect, s and v can be each independently 6 to 18; and c and d can be each independently 1 to 30. Other options for nonionic surfactants include those having the formula HOOC(CH₂)ᵢ-CH=CH- (CH₂)ₖ(CH₂CH₂O)_{z} H, where i, k are each independently 5 to 15; and z is 5 to 50. In another aspect, i and k are each independently 6 to 12; and z is 15 to 35.

The nonionic surfactant may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; which is hereby incorporated by reference or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991; hereby incorporated into the subject application by reference.

Illustrative examples of nonionic surfactants that can be used in the cleansing compositions disclosed herein include, but are not limited to, polyglycoside, cetyl alcohol, decyl glucoside, lauryl glucoside, octaethylene glycol monododecyl ether, n-octyl beta-d-thioglucopyranoside, octyl glucoside, oleyl alcohol, polysorbate, sorbitan, stearyl alcohol, or a combination thereof.

In an aspect, cationic surfactants can be used in the cleansing composition of the present application.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate.

Still other useful cationic surfactants include quaternized hydrolysates of silk, wheat, and keratin proteins, and it is within the scope of the cleansing composition to use mixtures of the aforementioned cationic surfactants.

If used, cationic surfactants will make up no more than 1.0% by weight of the cleansing composition. When present, cationic surfactants typically make up from 0.01 to 0.7%, and more typically, from 0.1 to 0.5% by weight of the cleansing composition, including any and all ranges and values subsumed therein.

The cleansing composition can additionally include up to 30% by weight skin benefit agents. The term "skin benefit agent" is defined as a substance which softens or improves the elasticity, appearance, and youthfulness of the skin (stratum corneum) by either increasing its water content, adding, or replacing lipids and other skin nutrients, or both, and keeps it soft by retarding the decrease of its water content. Included among the desirable skin benefit agents are emollients, including, for example, hydrophobic emollients, hydrophilic emollients, or blends thereof. Preferred benefit agents include moisturizers, emollients, sunscreens, and anti-aging compounds.

Desirably the optional skin benefit agents used in the cleansing composition disclosed herein include niacinamide (vitamin B₃), tocopherol (Vitamin E), aloe vera, alpha-hydroxy acids and esters, beta-hydroxy acids and esters, hydroxyethyl urea, polyhydroxy acids and esters, creatine, hydroquinone, t-butyl hydroquinone, mulberry, hyaluronic acid and salts thereof (including, but not limited to, Na+ and K+ salts of the same), extract, liquorice extract, resorcinol derivatives, or a combination thereof. For example, the skin benefit agent can be sodium hyaluronate. Such benefit agents, including sodium hyaluronate can be present in an amount of 0.0001 to 10%, for example, 0.001 to 6.5%, for example, 0.01 to 3.5%, and for example, 0.01% by weight, based on total weight of the cleansing composition including any and all ranges and values subsumed therein.

Further optional water-soluble skin benefit agents include acids, such as amino acids like arginine, valine or histidine. Other vitamins can be used such as vitamin B₂, picolinamide, panthenol (vitamin Bs), vitamin B₆, vitamin C, a combination thereof or the like. Derivatives (generally meaning something that has developed or been obtained from something else), and especially, water soluble derivatives of such vitamins can also be employed. For instance, vitamin C derivatives such as ascorbyl tetraisopalmitate, magnesium ascorbyl phosphate and ascorbyl glycoside may be used alone or in combination with each other. Niacinamide derivatives such as nicotinamide adenine dinucleotide (NADH) and nicotinamide adenine dinucleotide phosphate (NADPH) may be used alone or in combination with each other. Other skin benefit agents that can be used include 4-ethyl resorcinol, extracts like sage, aloe vera, green tea, sugar cane, citrus, grapeseed, thyme, chamomile, yarrow, cucumber, liquorice, rosemary extract, or a combination thereof. Electrolytes such as NaCl and/or KCI, MgCl₂ may also be used. The total amount of optional water-soluble benefit agents (including mixtures) when present in the composition disclosed herein can be 0.0001 to 10%, preferably, 0.001 to 6.5%, and most preferably, 0.01 to 3.5% by weight, based on total weight of the cleansing composition, including any and all values and ranges subsumed therein.

It is also within the scope of the cleansing composition to optionally include oil soluble benefit agents. Illustrative examples of the types of oil soluble benefit agents that can optionally be used in the cleansing composition disclosed herein include components like stearic acid, vitamins like vitamin A, D, E and K (and their oil soluble derivatives).

Other optional oil soluble benefit agents for use include resorcinols and resorcinol derivatives like 4-hexyl resorcinol, 4-phenylethyl resorcinol, 4-cyclopentyl resorcinol, 4-cyclohexyl resorcinol 4-isopropyl resorcinol or a combination thereof. Also, 5-substituted resorcinols like 4-cyclohexyl-5-methylbenzene-1,3-diol, 4-isopropyl-5-methylbenzene-1,3-diol, combination thereof or the like may be used. The 5-substituted resorcinols and their synthesis are described in commonly assigned U.S. Published Patent Application No. 2016/0000669A1.

Even other oil soluble benefit agents that can be used include omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 10-hydroxystearic acid (10HSA), 12-hydroxystearic acid (12HSA), petroselinic acid, conjugated linoleic acid, stearic acid, palmitic acid, lauric acid, terpineol, thymol essential components, the dissolution auxiliary selected from limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil, or a combination thereof. In this respect it is noted, that the amount of C6-C14 fatty acid preferably is below 3 wt%, more preferably below 1 wt%, even more preferably below 0.1 wt% and most preferably C6-C14 fatty acid is absent. It is preferred, that the level of lauric acid is is below 3 wt%, more preferably below 1 wt%, even more preferably below 0.1 wt% and most preferably lauric acid is absent. Low or even absence of these fatty acids reduces opacity. The present invention prefers to provide a clear composition.

Another optional oil soluble benefit agent that may be used is a retinoic acid precursor. The retinoic acid precursor can be retinol, retinal, retinyl ester, retinyl propionate, retinyl palmitate, retinyl acetate or a combination thereof. Retinyl propionate, retinyl palmitate, and combinations thereof are typically preferred. Still another retinoic acid precursor for use is hydroxyanasatil retinoate made commercially available under the name RETEXTRA^{®} as supplied by Molecular Design International. The same may be used in a combination with any of the oil soluble benefit agents described herein.

When an optional (i.e., 0.0 to 1.5% by weight) oil soluble benefit agent is used in the cleansing composition, it typically is present in an amount of 0.001 to 1.5% by weight of the overall cleansing composition including any and all values and ranges subsumed therein, and for example, 0.05 to 1.2% by weight, for example, 0.2 to 0.5% by weight of the total weight of the cleansing composition.

Other useful skin benefit agents include the following:
(a) silicone oils and modifications thereof such as linear and cyclic polydimethylsiloxanes; amino, alkyl, alkylaryl, and aryl silicone oils;
(b) fats and oils including natural fats and oils such as jojoba, soybean, sunflower, rice bran, avocado, almond, olive, sesame, persic, castor, coconut, and mink oils; cacao fat; beef tallow and lard; hardened oils obtained by hydrogenating the aforementioned oils; and synthetic mono, di and triglycerides such as myristic acid glyceride and 2-ethylhexanoic acid glyceride;
(c) waxes such as carnauba, spermaceti, beeswax, lanolin, and derivatives thereof;
(d) hydrophobic and hydrophilic plant extracts;
(e) hydrocarbons such as liquid paraffin, petrolatum, microcrystalline wax, ceresin, squalene, pristan and mineral oil;
(f) higher fatty acids such as lauric, myristic, palmitic, stearic, behenic, oleic, linoleic, linolenic, lanolic, isostearic, arachidonic and poly unsaturated fatty acids (PUFA);
(g) higher alcohols such as lauryl, cetyl, stearyl, oleyl, behenyl, cholesterol and 2-hexydecanol alcohol;
(h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol monolaurate, glycerol distearate, glycerol tristearate, alkyl lactate, alkyl citrate and alkyl tartrate;
(i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils;
(j) polyhydric alcohols, for example, glycerine, sorbitol, propylene glycol, and the like; and polyols such as the polyethylene glycols, examples of which are: Polyox WSR-205 PEG 14M, Polyox WSR-N-60K PEG 45M, or Polyox WSR-N-750, and PEG 7M;
(k) lipids such as cholesterol, ceramides, sucrose esters and pseudo-ceramides as described in European Patent Specification No. 556,957;
(l) vitamins, minerals, and skin nutrients such as milk, vitamins A, E, and K; vitamin alkyl esters, including vitamin C alkyl esters; magnesium, calcium, copper, zinc and other metallic components;
(m) sunscreens such as octyl methoxyl cinnamate (Parsol MCX) and butyl methoxy benzoylmethane (Parsol 1789);
(n) phospholipids; and
(o) anti-aging compounds such as alpha-hydroxy acids and beta-hydroxy acids.

Preferred skin benefit agents include fatty acids, hydrocarbons, polyhydric alcohols, polyols, and mixtures thereof, with emollients that include at least one C₁₂ to C₁₈ fatty acid, petrolatum, glycerol, glycerin, sorbitol, and/or propylene glycol being of particular interest in one or more embodiments. The agents may be added at an appropriate step during the process of making the cleansing compositions. Some benefit agents may be introduced as macro domains.

The cleansing composition can further comprise a humectant. The humectant can be present in an amount of 0.1 to 15% by weight, preferably 0.5 to 10% by weight, more preferably 0.75 to 8% by total weight of the cleansing composition, including any and all ranges and values subsumed therein. The humectant can be employed to assist in moisturization effects of the cleansing composition. Humectants are generally known as moisturizers that attract water from the air or deeper in the skin. Stated another way, humectants draw water into the skin, hair, or nails. The humectants can generally be polyhydric alcohol type materials. Typical polyhydric alcohols include glycerol (i.e., glycerine or glycerin), propylene glycol, dipropylene glycol, polypropylene glycol (e.g., PPG-9), polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol, or a combination thereof. Most preferred is glycerin, propylene glycol, dipropylene glycol, or a combination thereof. In an embodiment, the humectant can be propylene glycol, butylene glycol, dipropylene glycol, glycerin, triethylene glycol, erythritol, capryl glycol, hyaluronic acid, polypropylene glycol-7 proypyl heptyl ether, or a combination thereof.

Other optional ingredients like antioxidants, fragrances, perfumes, polymers, chelating agents, colorants, deodorants, dyes, enzymes, foam boosters, germicides, anti-microbials, lathering agents, pearlescers, skin conditioners, stabilizers, or superfatting agents, may be added in suitable amounts in the process of making the cleansing compositions.

Additional optional ingredients which may be present in the cleansing compositions are, for example: fragrances; sequestering and chelating agents such as tetrasodium ethylenediaminetetraacetate (EDTA), ethane hydroxyl diphosphonate (EHDP), and etidronic acid, aka 1-hydroxyethylidene diphosphonic acid (HEDP); coloring agents; opacifiers, and pearlizers such as zinc stearate, magnesium stearate, titanium dioxide (TiO₂), ethylene glycol monostearate (EGMS), ethylene glycol distearate (EGDS) or Lytron 621 (Styrene/Acrylate copolymer), and the like; pH adjusters; antioxidants, for example, butylated hydroxytoluene (BHT) and the like; stabilizers; suds boosters, such as for example, coconut acyl mono- or diethanol amides; ionizing salts, such as, for example, sodium chloride and sodium sulfate, and other ingredients such as are conventionally used in cleansing compositions. The total amount of such additional optional ingredients is typically from 0 to 10% by weight, more particularly from 0.1 to 5% by weight, based on the total weight of the cleansing composition, including any and all ranges and values subsumed therein.

The cleansing compositions disclosed herein can be used to deliver antimicrobial benefits. Antimicrobial agents that can be included to deliver these benefits include oligodynamic metals or compounds thereof. Preferred metals are silver, copper, zinc, gold, aluminum, or a. Silver is particularly preferred. In the ionic form it may exist as a salt or any compound in any applicable oxidation state. Preferred silver compounds are silver oxide, silver nitrate, silver acetate, silver sulfate, silver benzoate, silver salicylate, silver carbonate, silver citrate, silver phosphate, or a combination thereof, with silver oxide, silver sulfate and silver citrate being of particular interest in one or more embodiments. In at least one aspect, the silver compound is silver oxide. Oligodynamic metal or a compound thereof can be included in an amount of 0.0001 to 2%, preferably 0.001 to 1% by weight of the cleansing composition. Alternately an essential oil antimicrobial active may be included in the cleansing composition. Essential oil actives which can be included are terpineol, thymol, carvacol, (E) -2(prop-1-enyl) phenol, 2- propylphenol, 4-pentylphenol, 4-sec-butylphenol, 2-benzyl phenol, eugenol, or a combination thereof. Furthermore, preferred essential oil actives are terpineol, thymol, carvacrol, thymol, or a combination thereof, with the most preferred being terpineol or thymol, or a combination thereof. When present, essential oil actives can be included in an amount of 0.001 to 1%, preferably 0.01 to 0.5% by weight of the composition.

Even other ingredients which may be used include octopirox (piroctone), zinc pyrithione, chloroxylenol, triclosan, cetylpyridinium chloride, as well as silver compounds including silver oxide, nitrate, sulfate, phosphate, carbonate, acetate, benzoate, a combination thereof or the like. If used, these other components typically make up from 0.001 to 1.6% by weight of the overall cleansing composition including any and all values and ranges subsumed therein, and preferably, from 0.01 to 1.2% by weight of the overall cleansing composition including any and all values and ranges subsumed therein.

Preservatives can be used in the cleansing composition disclosed herein. When used, illustrative preservatives for use include sodium benzoate, iodopropynyl butyl carbamate (IPBC), caprylyl glycol (1,2-octane diol), phenoxyethanol, hydroxyacetophenone, ethylhexylglycerine, methyl paraben, propyl paraben, imidazolidinyl urea, sodium dehydroacetate, propanediol, dimethyl-dimethyl (DMDM) hydantoin, alkyl esters of para-hydroxybenzoic acid, organic acids and salts thereof, hydroxyacetophenone, climbazole, propionate salts, a variety of quaternary ammonium compounds, benzyl alcohol, or a combination thereof. Other preservatives suitable for use include sodium dehydroacetate, chlorophenesin, and decylene glycol. Preservatives can be employed in amounts of 0.01% to 2.0% by weight of the total weight of cleansing composition, including any and all values and ranges and values subsumed therein.

Fragrances, fixatives, opacifiers (like titanium dioxide or glycol distearate), and chelating agents may optionally be included in the cleansing composition. Possible chelating agents include, but are not limited to, ethylyene diaminetetraacetic acid (EDTA), diethylenetriaminepentaacetic acid (DTPA), ethylene diamine disuccinic acid (EDDS), pentasodium diethylenetriaminepentaacetate, trisodium N-(hydroxyethyl)-ethylenediaminetracetate, an acid form of EDTA, sodium thiocynate, trisodium salt of methylglycinediacetic acid, tetrasodium glutamate diacetate and phytic acid, preferably wherein the chelating agent is ethylene diaminetetraacetic acid (EDTA), diethylenetriaminepentaacetic acid (DTPA), ethylene diamine disuccinic acid (EDDS), or a combination thereof. Each of these substances may be present in an amount of about 0.03 to about 3% by weight of the overall cleansing composition including all values and ranges subsumed therein, preferably, about 0.1 to about 2.6% by weight, weight of the overall cleansing composition including all values and ranges subsumed therein.

The cleansing compositions can additionally comprise a viscosity adjusting agent. The viscosity adjusting agent can be a salt. The salt can comprise sodium chloride, potassium chloride, or a combination thereof. In an embodiment, the viscosity is a salt and the salt is sodium chloride, potassium chloride, or a combination thereof. The salt can be present in an amount of less than or equal to 1% of the overall cleansing composition.

The cleansing composition disclosed herein can further include a thickening agent. Particularly preferred thickening agents include an anionic polymer-based thickener, preferably, for example, a polyacrylate based polymer or copolymer, or a combination thereof. Polysaccharides can be used. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g., cellulose microfibrils, cellulose nanocrystals or microcrystalline cellulose).

Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

As mentioned, synthetic polymers are effective thickening agents. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as sodium acrylate/sodium acryloyldimethyl taurate and acryloyl dimethyltaurate/vinyl pyrrolidone copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

Sodium hydroxypropyl starch phosphate, aluminum starch octenylsuccinate, tapioca starch, maltodextrin, xanthan gum, agar gum, guar gum, carrageenan gum, alginate gum, hydroxypropyl cellulose, hydroxypropyl methylcellulose, ethylcellulose, sodium carboxy methylcellulose, cellulose, polyethylene glycol (e.g., polyethylene glycol diester stearic acid), or a combination thereof are further examples of thickening agents for use in the present cleansing compositions. Such thickening agents are commercially available from the Dow Chemical Company or the Hallstar Company.

Other optional ingredients include water soluble/dispersible polymers. These polymers can be cationic, anionic, amphoteric or nonionic types with molecular weights higher than 100,000 Dalton. They are known to increase the viscosity and stability of liquid personal cleansing formulation, to enhance in-use and after-use skin sensory properties, and to enhance lather creaminess and lather stability. When present, the total amount of such polymers commonly ranges from 0.1 to 10% by weight of the personal cleansing formulation.

Examples of water soluble or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn^{®} 28, Aculyn^{®} 22 or Carbopol ^{®}Aqua SF1; cationic polymer such as modified polysaccharides including cationic guar available from Rhone Poulenc under the trade name Jaguar C13S, Jaguar C14S, Jaguar C17, or Jaguar C16; cationic modified cellulose such as UCARE Polymer JR 30 or JR 40 from Amerchol; N-Hance^{®} 3000, N-Hance^{®} 3196, N-Hance^{®} GPX 215 or N-Hance^{®} GPX 196 from Hercules; synthetic cationic polymer such as Merquat^{®} 100, Merquat^{®} 280, Merquat^{®} 281 and Merquat^{®} 550 sold by Nalco; cationic starches such as StaLok^{®} 100, 200, 300 and 400 sold by Staley Inc.; cationic galactomannans such as Galactasol^{®} 800 series by Henkel, Inc.; Quadrosoft^{®} LM-200; and Polyquaternium-24. Also suitable are high molecular weight polyethylene glycols such as Polyox^{®} WSR-205 (PEG 14M), Polyox^{®} WSR-N-60K (PEG 45), and Polyox^{®} WSR-301 (PEG 90M).

The cleansing compositions herein described can be produced by preparative techniques. In one very general methodology, to a heated aqueous phase of water, is added melted fatty acid, followed by caustic (to neutralize the fatty acid and form soap), synthetic detergents and cosolvents; the remaining ingredients are added, as appropriate, as the product is cooled to room temperature.

The cleansing compositions can be provided in a variety of different product forms including, for example, hand, face and body washes, shower gels, bars, and the like, e.g., antimicrobial product forms including, for example, hand, face and body washes, shower gels, bars, and the like. The formulations may be provided in bottles, pump dispensers, tubes, sachets, or other packaging suitable for the product form.

In use, the cleansing compositions are diluted, as needed, to form aqueous cleansing compositions that are applied to the skin for contact times less than 1 minute, more particularly 30 seconds or less (with contact times of 10 to 30 seconds being of interest with respect to contact times of a moderate to a relatively long duration and contact times of 10 seconds or less being of interest with respect to contact times of short to moderate duration) and thereafter are removed from the skin, typically by rinsing with water. The cleansing compositions can be diluted before, after or simultaneous with its being placed on the skin, with dilution typically occuring by the formulation being worked into a lather in the hands or on an applicator, such as a facecloth, sponge or pouf.

Inorganic salt is an optional but often desired ingredient to aid in composition thickening. Salts that may be used include NaCl, KCI, MgCl₂, CaCl₂, combinations thereof, or the like. Typically, the inorganic salt makes up 0 to 10% by weight, and preferably, 0.001 to 12% by weight, more preferably, from 0.05 to 4.5% by weight, and even more preferably, 0.09 to 2% by weight of the concentrated cleansing composition, including any and all ranges and values subsumed therein.

Polymeric viscosity aids are an optional but often desired ingredient in the cleansing composition. Preferred polymers are those generally classified as high molecular weight ethoxylated fatty acid esters. Illustrative examples include PEG 120 methyl glucose dioleate, PEG 18 glyceryloleate/cocoate, PEG 150 pentaerythritol tetrastearate, or combinations thereof, or the like. One polymeric viscosity aid is PEG 150 pentaerythritol tetrastearate which is sold under the VERSATHIX^{™} name by Croda. When used, such aids make up from 0.001 to 0.8%, and preferably, from 0.001 to 0.5%, and most preferably, from 0.001 to 0.3% by weight of the cleansing composition, including any and all ranges and values subsumed therein.

Adjusters to modify/buffer the pH may be used. Such pH adjusters include triethylamine, NaOH, KOH, H₂SO₄, HCl, C₆H₈O₇ (i.e., citric acid), C₃H₆O₃ (i.e., lactic acid), or a combination thereof. The pH adjusters are added at amounts to yield the desired final pH. The pH adjusters can include various alpha-hydroxy acids or a combination thereof. For example, the pH adjusters can comprise the alpha-hydroxy acids of citric acid, glycolic acid, lactic acid, malic acid, tartaric acid, or a combination thereof. The pH values can be assessed with commercial instrumentation such as a pH meter made commercially available from Thermo Scientific^{®}. The pH adjusters can be present in an amount of 0.1 to 1.0% by weight of the cleansing composition, including any and all ranges and values subsumed therein, for example, 0.1 to 0.75% by weight of the cleansing composition, including any and all ranges and values subsumed therein.

The pH of the composition is assessed by using conventional instrumentation such as a pH meter made commercially available from Thermo Scientific^{®}. A pH of the cleansing composition can be 3 to 9, preferably, 4 to 8, more preferably, 5 to 7.

The cleansing compositions disclosed herein typically contain water in an amount of 20 to 95% by weight, more particularly 50 to 93% by weight, more specially, 60 to 90% by weight, based on the total weight of the cleansing composition, including any and all ranges and values subsumed therein. Such water contents are representative of a relatively broad range of formulations, including both concentrated and non-concentrates products, with formulations having water contents of 20 to less than 50% by weight of water being typical of concentrated products.

Viscosity, unless noted otherwise, was measured with a Discovery HR-2 Rheometer using sand blasted plates with a 1000 micron gap at 25°C and 30 second intervals and a shear rate of 4 s⁻¹. The cleansing compositions disclosed herein had a viscosity change of more than 40% at 4s-1 at a dilution of 2 parts product to 1 part water using a Discovery HR-2 Rheometer using sand blasted plates having a 1000 micron gap, at 25°C and 30 second intervals and a shear rate of 4 s⁻¹. Not wishing to be bound by theory, it is hypothesized that the rapid drop in viscosity on dilution reflects a more rapid breakdown of the worm-like micelles to spherical micelles facilitated by the increased levels of short chain isethionate surfactants which allows for the lather profile of the inventive composition to be similar to a typical sulfate-based cleansing composition as shown in Table 2.

**Table 2 - Typical SLES based cleansing composition**

| Material | %, 100% |
|---|---|
| Water | 79.0 |
| Polymeric Thickener | 0-6.0 |
| Electrolyte | 0-4.0 |
| Sodium Laureth Sulfate | 12.3 |
| Cocamide MEA | 1.0 |
| Preservative | 0.5 |
| Benefit agents | 0.5-5.0 |

The cleansing composition is essentially sulfate free. Preferably, the cleansing composition is sulfate free.

The cleansing composition is essentially 1,4-dioxane free. Preferably, the cleansing composition is 1,4-dioxane free.

Furthermore, the cleansing composition can be transparent down to a temperature of 5°C and have a viscosity drift of less than 30% after exposure for 2 weeks 50°C. This result was surprising and unexpected when replacing a small portion of the first anionic surfactant with shorter chains such as C8 or C10 isethionate or C9 isethionate.
All amounts are by weight of the final composition, unless otherwise specified.
In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight, etc.). "Combination is inclusive of blends, mixtures, alloys, reaction products, and the like. Furthermore, the terms "first", "second", and the like herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term it modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects.

For the avoidance of doubt the word "comprising" is intended to mean "including" but not necessarily "consisting of" or "composed of." In other words, the listed steps, options, or alternatives need not be exhaustive.

The disclosure of the invention as found herein is to be considered to cover all aspects as found in the claims.
Unless otherwise specified, numerical ranges expressed in the format "from x to y" are understood to include x and y.
All percentages and ratios contained herein are calculated by weight unless otherwise indicated.

### Examples

The following examples are merely illustrative of the cleansing composition disclosed herein and are not intended to limit the scope hereof.

Table 3 lists the composition of a cleansing composition according to the inventive compositions disclosed herein.

**Table 3: Inventive Cleansing Composition**

| Material | %, 100% |
|---|---|
| Water | 85 |
| Sodium gluconate | 0.05 |
| Thickener | 0.50 |
| Isethionate | 3.00 |
| CAPB* | 6.00 |
| Preservative | 1.30 |
| Benefit agents | 0.5-5.0 |

| | |
|---|---|
| *CAPB=cocoamidopropyl betaine | |

In this inventive sample (Inventive 1), some of the C12 chains of the isethionate were replaced by C9 chains as noted in Table 4. Comparative Example 1 contained typical isethionate chain lengths and Comparative Example 2 contained some amount of C9 isethionate, but the total fraction of short chain lengths still remained below 11%.

**Table 4 -**

| | Inventive 1 | Comparative 1 | Comparative 2 |
|---|---|---|---|
| | + 10% C9 isethionate | + 0% C9 isethionate | + 5% C9 isethionate |
| C8, C9, C10 isethionat e | 0.435% [0.08%C8/0.30%C9/0.05%C1 0] | 0.15% [0.09%C8/0.06 % C10] | 0.293% [0.09%C8/0.15%C9/0.05%C1 0] |
| C12, C14 isethionat e | 2.54% | 2.83% | 2.69% |
| Other chains | 0.02% | 0.02% | 0.02% |
| Fraction of Short | 14.79% | 5.03% | 9.82% |
| Chain Lengths | | | |
| Drop in viscosity on dilution (1 part water 2 parts product) | 63% | 30% | 11% |

**Table 5 - Inventive Cleansing Composition**

| Material | %, 100% |
|---|---|
| Water | 87.2 |
| Sodium Benzoate | 0.5 |
| NaCl | 1 |
| Isethionate | 4 |
| CAPB | 6 |
| CAPG* | 0.30 |
| Fragrance | 1 |

| | |
|---|---|
| CAPG=caprylyl glycol | |

In these inventive samples (Inventive 2 and Inventive 3), some of the C12 chains of the isethionate were replaced by C8 and/or C10 chains as noted in Table 6. Comparative Example 2 contained typical isethionate chain lengths.

**Table 6 -**

| | Inventive 2 | Inventive 3 | Comparative 2 |
|---|---|---|---|
| | + 10% C8 isethionate | + 5% C8 / 5% C10 isethionate | + 0% C 8 - C10 isethionate |
| C8, C9, C10 isethionate | 0.63% [0.55%C8/0.08%C10] | 0.63% [0.34%C8/0.29% C10] | 0.256% [0.15%C8/0.102%C10] |
| C12, C14 isethionate | 3.36% | 3.36% | 3.73% |
| Other chains | 0.02% | 0.02% | 0.02% |
| Fraction of Short Chain Lengths | 15.8% | 15.8% | 6.4% |
| Drop in viscosity on dilution (1 part water 2 parts product) | 77% | 81% | 15% |

As can be seen from Table 4 and Table 6, Inventive examples 1, 2 and 3 contained a higher fraction of short chain lengths, where short chain lengths refer to C8, C9, and C10 chain lengths. A drop in viscosity greater than 40% when the composition was diluted with one part water was observed at 4s-1 at a dilution of 2 parts product to 1 part water using a Discovery HR-2 Rheometer using sand blasted plates having a 1000 micron gap, at 25°C and 30 second intervals and a shear
rate of 4 s⁻¹. A milder composition was observed and parity in terms of foaming with a standard sodium lauryl ether sulfate-based composition was achieved. Comparative Example 1 and Comparative Example 2 where the fraction of short chains was below 11% did not show a corresponding drop in viscosity. The examples in Table 4 and Table 6 indicated that by replacing a portion of the C12 chains of isethionate with C8, C10 and/or C9 isethionate a rapid drop in viscosity on dilution can be achieved allowing a lather profile similar to a typical sulfate-based cleansing formulation. Lathering of the formulations was assessed in-hand.

Furthermore, all Inventive examples 1, 2 and 3 where a portion of C12-C14 chains were replaced with short chains were exposed to a temperature of 50°C for a period of two weeks. Surprisingly it was found that all compositions had a viscosity drift of less than 30% and remained clear after this two week period.

## Claims

1. A cleansing composition, comprising:
3 to 20% by weight of a total amount of surfactant, preferably, 5 to 18% by weight of a total amount of surfactant, more preferably, 5 to 15% by weight of a total amount of surfactant, based on the total weight of the cleansing composition, wherein the surfactant comprises:
• an anionic surfactant comprising a first anionic surfactant comprising:
∘ 11 to 30% by weight C8 to C10 chains, preferably, 12 to 25% by weight C8 to C10 chains, more preferably, 13 to 20% by weight C8 to C10 chains, based on the total weight of the anionic surfactant;
∘ 70 to 89% by weight C12 to C14 chains, based on the total weight of the anionic surfactant; and
∘ less than or equal to 0.5% by weight C16 to C18 chains, based on the total weight of the anionic surfactant; and
• a zwitterionic surfactant;
wherein a weight ratio of anionic surfactant to zwitterionic surfactant is 1:4 to 1:1, preferably wherein the weight ratio of anionic surfactant to zwitterionic surfactant is 1:2 to 2:3; and wherein the first anionic surfactant comprises isethionate.

2. The cleansing composition of Claim 1, wherein the isethionate comprises sodium lauroyl isethionate.

3. The cleansing composition of Claim 1 or Claim 2, wherein the first anionic surfactant is present in an amount of greater than or equal to 85% by weight of the anionic surfactant.

4. The cleansing composition of any of the preceding claims, wherein the anionic surfactant further comprises a second anionic surfactant, wherein the second anionic surfactant is present in an amount of less than or equal to 15% by weight of the anionic surfactant.

5. The cleansing composition of Claim 4, wherein the second anionic surfactant comprises sodium lauroyl glycinate, sodium cocoyl glycinate, sodium lauroyl glutamate, sodium cocoyl glutamate, sodium lauryl sarcosinate, alpha olefin sulfonate (AOS), or a combination thereof.

6. The cleansing composition of any of the preceding claims, wherein the zwitterionic surfactant comprises lauryl betaine, cocobetaine, (carboxymethyl) dimethyl-3-[(1-oxododecyl) amino] propylammonium hydroxide, coco alkyldimethyl betaine, (carboxymethyl) dimethyloleylammonium hydroxide, cocoamidopropyl betaine, (carboxylatomethyl) dimethyl(octadecyl)ammonium, cocamidopropyl hydroxysultaine, laurylamidopropyl betaine, lauryl hydroxysultaine, or a combination thereof.

7. The cleansing composition of any of the preceding claims, wherein the C14 chains are present in an amount of less than or equal to one half of the sum of C12 and C14 chains present in the first anionic surfactant, preferably wherein the C14 chains are present in an amount of less than or equal to one third of the sum of C12 and C14 chains present in the first anionic surfactant.

8. The cleansing composition of any of the preceding claims, further comprising a viscosity adjusting agent.

9. The cleansing composition of Claim 8, wherein the viscosity adjusting agent is a salt, preferably wherein the salt comprises sodium chloride, potassium chloride, a combination thereof.

10. The cleansing composition of any of the preceding claims, wherein the cleansing composition is essentially sulfate free, preferably wherein the cleansing composition is sulfate free.

11. The cleansing composition of any of the preceding claims, wherein the cleansing composition is essentially 1,4-dioxane free, preferably wherein the cleansing composition is 1,4-dioxane free.

12. The cleansing composition of any of the preceding claims, wherein the cleansing composition has a change in viscosity of greater than 40% when diluted with 1 part water.

13. The cleansing composition of any of the preceding claims, wherein a viscosity of the cleansing composition changes by less than or equal to 30% after a two-week time period stored at 50°C measured using a Discovery HR-2 Rheometer using sand blasted plates having a 1000 micron gap, at 25°C and 30 second intervals and a shear rate of 4 s⁻¹.

## Patentansprüche

1. Reinigungszusammensetzung, umfassend:
3 bis 20 Gew.-% der Gesamtmenge an Tensid, vorzugsweise 5 bis 18 Gew.-% der Gesamtmenge an Tensid, bevorzugter 5 bis 15 Gew.-% der Gesamtmenge an Tensid, bezogen auf das Gesamtgewicht der Reinigungszusammensetzung, wobei das Tensid umfasst:
• ein anionisches Tensid, das ein erstes anionisches Tensid umfasst, umfassend:
∘ 11 bis 30 Gew.-% C8- bis C10-Ketten, vorzugsweise 12 bis 25 Gew.-% C8- bis C10-Ketten, bevorzugter 13 bis 20 Gew.-% C8- bis C10-Ketten, bezogen auf das Gesamtgewicht des anionischen Tensids;
∘ 70 bis 89 Gew.-% C12- bis C14-Ketten, bezogen auf das Gesamtgewicht des anionischen Tensids; und
∘ weniger als oder gleich 0,5 Gew.-% C16- bis C18-Ketten, bezogen auf das Gesamtgewicht des anionischen Tensids; und
• ein zwitterionisches Tensid;
wobei das Gewichtsverhältnis von anionischem Tensid zu zwitterionischem Tensid 1:4 bis 1:1 beträgt, wobei das Gewichtsverhältnis von anionischem Tensid zu zwitterionischem Tensid vorzugsweise 1:2 bis 2:3 beträgt; und
wobei das erste anionische Tensid Isethionat umfasst.

2. Reinigungszusammensetzung nach Anspruch 1, wobei das Isethionat Natriumlauroylisethionat umfasst.

3. Reinigungszusammensetzung nach Anspruch 1 oder Anspruch 2, wobei das erste anionische Tensid in einer Menge von mehr als oder gleich 85 Gew.-% des anionischen Tensids vorliegt.

4. Reinigungszusammensetzung nach einem der vorhergehenden Ansprüche, wobei das anionische Tensid ferner ein zweites anionisches Tensid umfasst, wobei das zweite anionische Tensid in einer Menge von weniger als oder gleich 15 Gew.-% des anionischen Tensids vorliegt.

5. Reinigungszusammensetzung nach Anspruch 4, wobei das zweite anionische Tensid Natriumlauroylglycinat, Natriumcocoylglycinat, Natriumlauroylglutamat, Natriumcocoylglutamat, Natriumlaurylsarcosinat, Alpha-Olefinsulfonat (AOS) oder eine Kombination davon umfasst.

6. Reinigungszusammensetzung nach einem der vorhergehenden Ansprüche, wobei das zwitterionische Tensid umfasst: Laurylbetain, Cocobetain, (Carboymethyl)dimethyl-3[(1-oxododecyl)amino]propylammoniumhydroxid, Cocoalkyldimethylbetain, (Carboxymethyl)dimethyloleylammoniumhydroxid, Cocoamidopropylbetain, (Carboxylatomethyl)dimethyl(octadecyl)ammonium, Cocoamidopropylhydroxysultain, Laurylamidopropylbetain, Laurylhydroxysultain oder eine Kombination davon.

7. Reinigungszusammensetzung nach einem der vorhergehenden Ansprüche, wobei die C14-Ketten in einer Menge von weniger als oder gleich der Hälfte der Summe der im ersten anionischen Tensid vorhandenen C12- und C14-Ketten vorliegen, wobei die C14-Ketten vorzugsweise in einer Menge von weniger als oder gleich einem Drittel der Summe der im ersten anionischen Tensid vorhandenen C12- und C14-Ketten vorliegen.

8. Reinigungszusammensetzung nach einem der vorhergehenden Ansprüche, die ferner ein Viskositätsregulierungsmittel umfasst.

9. Reinigungszusammensetzung nach Anspruch 8, wobei das Viskositätsregulierungsmittel ein Salz ist, wobei das Salz vorzugsweise Natriumchlorid, Kaliumchlorid oder eine Kombination davon umfasst.

10. Reinigungszusammensetzung nach einem der vorhergehenden Ansprüche, wobei die Reinigungszusammensetzung im Wesentlichen sulfatfrei ist, wobei die Reinigungszusammensetzung vorzugsweise sulfatfrei ist.

11. Reinigungszusammensetzung nach einem der vorhergehenden Ansprüche, wobei die Reinigungszusammensetzung im Wesentlichen frei von 1,4-Dioxan ist, wobei die Reinigungszusammensetzung vorzugsweise frei von 1,4-Dioxan ist.

12. Reinigungszusammensetzung nach einem der vorhergehenden Ansprüche, wobei die Reinigungszusammensetzung bei Verdünnung mit 1 Teil Wasser eine Viskositätsänderung von mehr als 40% aufweist.

13. Reinigungszusammensetzung nach einem der vorhergehenden Ansprüche, wobei sich die Viskosität der Reinigungszusammensetzung nach einer zweiwöchigen Lagerung bei 50°C um weniger als oder gleich 30% ändert, gemessen mit einem Discovery HR-2-Rheometer unter Verwendung sandgestrahlter Platten mit einem Spalt von 1000 Mikrometern, bei 25°C und in Intervallen von 30 Sekunden sowie einer Scherrate von 4 s⁻¹.

## Revendications

1. Composition de nettoyage, comprenant :
3 à 20 % en poids d'une quantité totale de tensioactif, de préférence, 5 à 18 % en poids d'une quantité totale de tensioactif, de manière mieux préférée, 5 à 15 % en poids d'une quantité totale de tensioactif, sur la base du poids total de la composition de nettoyage, dans laquelle le tensioactif comprend :
• un tensioactif anionique comprenant un premier tensioactif anionique comprenant :
∘ 11 à 30 % en poids de chaînes C8 à C10, de préférence 12 à 25 % en poids de chaînes C8 à C10, de manière mieux préférée, 13 à 20 % en poids de chaînes C8 à C10, sur la base du poids total du tensioactif anionique ;
∘ 70 à 89 % en poids de chaînes C12 à C14, sur la base du poids total du tensioactif anionique ; et
∘ 0,5 % en poids ou moins de chaînes C16 à C18, sur la base du poids total du tensioactif anionique ; et
• un tensioactif zwitterionique ;
dans laquelle un rapport pondéral du tensioactif anionique sur le tensioactif zwitterionique est de 1 : 4 à 1 : 1, de préférence dans laquelle le rapport pondéral du tensioactif anionique sur le tensioactif zwitterionique est de 1 : 2 à 2 : 3 ; et
dans laquelle le premier tensioactif anionique comprend l'iséthionate.

2. Composition de nettoyage selon la revendication 1, dans laquelle l'iséthionate comprend le sodium lauroyl iséthionate.

3. Composition de nettoyage selon la revendication 1 ou la revendication 2, dans laquelle le premier tensioactif anionique est présent en une quantité supérieure ou égale à 85 % en poids du tensioactif anionique.

4. Composition de nettoyage selon l'une quelconque des revendications précédentes, dans laquelle le tensioactif anionique comprend en outre un deuxième tensioactif anionique, dans laquelle le deuxième tensioactif anionique est présent en une quantité inférieure ou égale à 15 % en poids du tensioactif anionique.

5. Composition de nettoyage selon la revendication 4, dans laquelle le deuxième tensioactif anionique comprend le sodium lauroyl glycinate, le sodium cocoyl glycinate, le sodium lauroyl glutamate, le sodium cocoyl glutamate, le sodium lauryl sarcosinate, l'apha-oléfine sulfonate (AOS), ou une combinaison de ceux-ci.

6. Composition de nettoyage selon l'une quelconque des revendications précédentes, dans laquelle le tensioactif zwitterionique comprend la lauryl bétaïne, la cocobétaïne, l'hydroxyde de (carboxyméthyl) diméthyl-3-[(1-oxododécyl)amino]propylammonium, la cocoalkyldiméthyl bétaïne, l'hydroxyde de (carboxyméthyl) diméthyloléylammonium, la cocamidopropyl bétaïne, le (carboxylatométhyl) diméthyl(octadécyl)-ammonium, la cocamidopropyl hydroxysultaïne, la laurylamidopropyl bétaïne, la lauryl hydroxysultaïne, ou une combinaison de ceux-ci.

7. Composition de nettoyage selon l'une quelconque des revendications précédentes, dans laquelle les chaînes C14 sont présentes en une quantité inférieure ou égale à la moitié de la somme des chaînes C12 et C14 présentes dans le premier tensioactif anionique, de préférence dans laquelle les chaînes C14 sont présentes en une quantité inférieure ou égale à un tiers de la somme des chaînes C12 et C14 présentes dans le premier tensioactif anionique.

8. Composition de nettoyage selon l'une quelconque des revendications précédentes, comprenant en outre un agent régulateur de viscosité.

9. Composition de nettoyage selon la revendication 8, dans laquelle l'agent régulateur de viscosité est un sel, de préférence dans laquelle le sel comprend le chlorure de sodium, le chlorure de potassium ou une combinaison de ceux-ci.

10. Composition de nettoyage selon l'une quelconque des revendications précédentes, la composition de nettoyage étant sensiblement exempte de sulfate, de préférence la composition de nettoyage étant exempte de sulfate.

11. Composition de nettoyage selon l'une quelconque des revendications précédentes, la composition de nettoyage étant sensiblement exempte de 1,4-dioxane, de préférence la composition de nettoyage étant exempte de 1,4-dioxane.

12. Composition de nettoyage selon l'une quelconque des revendications précédentes, la composition de nettoyage ayant une variation de viscosité supérieure à 40 % lorsqu'elle est diluée avec 1 partie d'eau.

13. Composition de nettoyage selon l'une quelconque des revendications précédentes, dans laquelle une viscosité de la composition de nettoyage varie de 30 % ou moins après une durée de 2 semaines stockée à 50°C, mesurée en utilisant le rhéomètre Discovery HR-2 en utilisant des plaques sablées ayant un espace de 1000 microns, à 25°C et à des intervalles de 30 secondes et un taux de cisaillement de 4 s⁻¹.
